# EUROPEAN PATENT APPLICATION

(11) **EP 3 581 924 A1**
(43) Date of publication of application: **18.12.2019**
(21) Application number: 19179647.3
(22) Date of filing: 12.06.2019
(51) Int. Cl.: G01N 27/327

(54) **MEASUREMENT APPARATUS AND MEASUREMENT SYSTEM**

(30) Priority: 12.06.2018 JP 2018112170; 10.06.2019 JP 2019108231
(71) Applicant: ARKRAY, Inc., Kyoto-shi, Kyoto 601-8045 (JP)
(72) Inventor: Gokuden, Risa, Kyoto, 602-0008 (JP); Kaneda, Hisashi, Kyoto, 602-0008 (JP)
(74) Representative: Hoffmann Eitle

(57) **Abstract**

A measurement apparatus for measuring a target substance in a sample adhered to a sensor includes a plurality of conductive portions that includes a first conductive portion group extending in an insertion direction of the sensor, and a second conductive portion group extending in a direction intersecting with the insertion direction. In the inserted state, the first conductive portion group is configured to contact with electrode pads corresponding to the first conductive portion group, and the second conductive portion group is configured to contact with electrode pads other than the electrode pads corresponding to the first conductive portion group.

## Description

### BACKGROUND

### 1. Field

The present invention relates to a measurement apparatus and a measurement system.

### 2. Description of the Related Art

Various measurement apparatuses for measuring one's glucose level (a value of glucose concentration in blood) are commercially available. For example, a self-monitoring of blood glucose (SMBG) apparatus for performing SMBG has been known as a glucose level measuring apparatus. With an SMBG apparatus, blood collected from a fingertip or the like using a puncture device is spotted (caused to adhere) onto a sample holder mounted to the measurement apparatus and a glucose level is measured.

There is a sample holder including a first loaded portion to be inserted into a first receiving portion of an analyzer and a second loaded portion to be inserted into a second receiving portion which is provided in the analyzer or another analyzer and which differs from the first receiving portion (for example, Patent Document 1). With the sample holder, a pair of first pads formed on the first loaded portion and a pair of second pads formed on the second loaded portion are arranged in the width direction of the sample holder.

<Patent Document 1> Japanese Patent Application Laid-open No. 2013-092386

### SUMMARY

Due to reasons such as enabling a plurality of measurement items to be measured using one sample holder, the number of electrodes mounted to a sample holder has been increasing. As the number of electrodes increases, the number of pads for electrically connecting the respective electrodes to a measurement apparatus or an analyzer also increases. When a plurality of pads is arranged side by side in a width direction of a sample holder as in conventional art, a size of the sample holder increases.

However, there is a downsizing trend of test pieces from the perspectives of simplifying management, reducing manufacturing cost, and the like. Therefore, an increase in the size of sample holders in the width direction due to an increase in the number of electrodes is not favorable.

An embodiment of the present invention has been made in consideration of the circumstances described above and an object thereof is to provide a measurement apparatus capable of downsizing a sensor.

One of embodiments is a measurement apparatus to measure a target substance in a sample adhered to a sensor. The measurement apparatus includes:
an insertion opening used to insert an inserting portion of the sensor within the measurement apparatus, the inserting portion including a plane having a plurality of electrode pads; and
a plurality of conductive portions configured to contact with the plurality of electrode pads in an inserted state of the sensor indicating a state that the inserting portion of the sensor is inserted within the measurement apparatus,
wherein the plurality of conductive portions includes a first conductive portion group extending in an insertion direction of the sensor, the insertion direction indicating a direction of the sensor when the inserting portion is inserted within the measuring apparatus passing through the insertion opening, and a second conductive portion group extending in a direction intersecting with the insertion direction, and
wherein in the inserted state, the first conductive portion group is configured to contact with electrode pads corresponding to the first conductive portion group among the plurality of electrode pads, and the second conductive portion group is configured to contact with electrode pads other than the electrode pads corresponding to the first conductive portion group among the plurality of electrode pads.

In the measurement apparatus of the one of embodiments, the plurality of conductive portions may be configured below. For example, the plurality of conductive portions may be configured that each of the plurality of conductive portions has one end and other end, the one end is supported in the measuring apparatus, and the other end is contactable the plurality of electrode pads formed on the plane of the inserting portion in the inserted state, and, in the inserted state, each of the plurality of conductive portions has a farthest portion apart from the plane in the normal direction of the plane, a distance in the normal direction between the plane and the farthest portion is shorter than a given distance. In the one of embodiments, the plurality of conductive portions may apply configuration that in the inserted state, a level of surface of each of the plurality of electrode pads in the horizontal direction of the plane is same, and a distance in the normal direction of the plane between the surface and the farthest portion is within 1.5 times a thickness of each of the plurality of conductive portions. Further, in the one of embodiments, the plurality of conductive portions may apply configuration that in the inserted state, each of the plurality of conductive portions is arranged on a plane that is parallel to the plane included in the inserting portion.

In the one of embodiments, the measurement apparatus may apply configuration that the second conductive portion group are configured to contact with electrode pads other than the contact pads contacting with the first conductive portion group in the inserted state among the plurality of electrode pads.

In the one of embodiments, the measurement apparatus may further include a determining portion configured to determine, in the inserted state, whether a first conductive portion in the first conductive portion group and a second conductive portion in the second conductive portion group are in contact with a same electrode among the plurality of contact pads or in contact with two different electrode pads among the plurality of electrode pads. For example, the determining portion may be configured to determine whether or not a change in a current occurs by applying a voltage between the first conductive portion and the second conductive portion.

In the one of embodiments, the measurement apparatus may further include a determining portion configured to determine, in the inserted state, whether two second conductive portions in the second conductive portion group are in contact with a same electrode pad among the plurality of contact pads or in contact with two different electrode pads among the plurality of electrode pads. For example, the determining portion is configured to determine whether or not a change in a current occurs by applying a voltage between the two second conductive portions.

Another one of the embodiments is a measurement system. The measurement system includes:
a sensor to which a sample is to be adhered; and
a measurement apparatus for measuring a target substance in the sample,
wherein the measurement apparatus includes:
   an insertion opening used to insert an inserting portion of the sensor within the measurement apparatus, the inserting portion including a plane having a plurality of electrode pads; and
   a plurality of conductive portions configured to contact with the plurality of electrode pads in an inserted state of the sensor indicating a state that the inserting portion of the sensor is inserted within the measurement apparatus,
   wherein the plurality of conductive portions includes a first conductive portion group extending in an insertion direction of the sensor, the insertion direction indicating a direction of sensor when the inserting portion is inserted within the measuring apparatus passing through the insertion opening, and a second conductive portion group extending in a direction intersecting with the insertion direction, and
   wherein in the inserted state, the first conductive portion group is configured to contact with electrode pads corresponding to the first conductive portion group among the plurality of electrode pads, and the second conductive portion group is configured to contact with electrode pads other than the electrode pads corresponding to the first conductive portion group among the plurality of electrode pads.

Moreover, an embodiment of the measurement apparatus or the measurement system may apply a configuration that a tip portion of conductive portions belonging to the second conductive portion group is oriented in the insertion direction. An embodiment of the measurement apparatus or the measurement system may apply a configuration that the second conductive portion group is arranged such that, during insertion of the inserting portion, the second conductive portion group and the plurality of electrode pads rub against each other, and, when the inserting portion reaches a given position, the second conductive portion group contacts with electrode pads corresponding to the second conductive portion group. An embodiment of the measurement apparatus or the measurement system may apply configuration that at least one of the plurality of conductive portions has an intermediate portion between a base end portion and a tip portion thereof, and wherein the intermediate portion slopes downward so as to intersect the insertion direction.

### BRIEF DESCRIPTION OF THE DRAWINGS

FIG. 1 is a diagram showing a configuration example of a measurement system according to an embodiment;
FIG. 2 is a plan view showing an example of a sensor according to the embodiment;
FIG. 3 is a sectional view showing an example of a sensor according to the embodiment;
FIG. 4 is an exploded perspective view showing an example of a sensor according to the embodiment;
FIG. 5 is a plan view showing an enlargement of a main body portion of a sensor;
FIG. 6A is a plan view schematically showing a state where a connecting portion is fitted with a receiving portion, and FIG. 6B is a side view schematically showing a left side surface (a lower side in FIG. 6A) of the connecting portion and the receiving portion shown in FIG. 6A;
FIG. 7 shows a cross section taken along a dashed-dotted line B-B in FIG. 6A;
FIG. 8 shows a configuration example of a measurement apparatus;
FIG. 9 is a flow chart showing an example of operations of a measurement apparatus;
FIG. 10 shows a first modification of a measurement apparatus;
FIG. 11 shows a second modification of a measurement apparatus;
FIGS. 12A and 12B show configuration examples of a sensor and a measurement apparatus according to a second embodiment;
FIG. 13 is a flow chart showing an example of processes related to a determination of specifications of a sensor;
FIG. 14 shows modifications of a sensor and a measurement apparatus according to the second embodiment;
FIG. 15 is a plan view schematically showing a state where a connecting portion and a receiving portion are fitted with each other according to a third embodiment; and
FIG. 16 is an explanatory diagram of a connector pin according to a fourth embodiment.

### DESCRIPTION OF THE PREFERRED EMBODIMENTS

Hereinafter, embodiments of the present invention will be described with reference to the drawings. It is to be understood that configurations of the embodiments described below are illustrative and that the present invention is not limited to the configurations of the embodiments described below.

### First Embodiment

### <Outline of Measurement System>

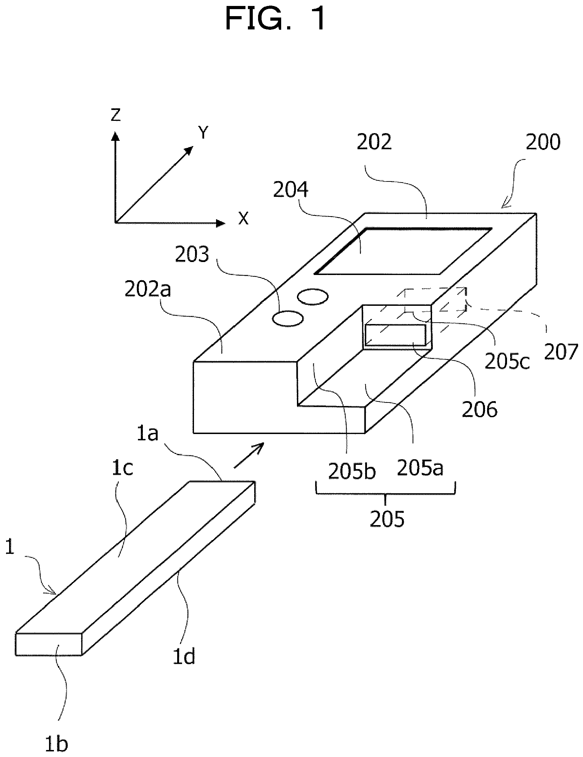

FIG. 1 is a diagram showing a configuration example of a measurement system according to an embodiment. The measurement system includes a sensor 1 and a measurement apparatus 200. The measurement apparatus 200 measures a target substance inside a sample by an electrochemical method using the sensor 1. The measurement apparatus 200 includes a housing 202, an operation button 203, a display panel 204, a stepped portion 205, and an insertion opening (also referred to as a slot) 206 for the sensor 1.

For example, the housing 202 is formed in a thin rectangular parallelepiped shape of which a dimension in a height direction (a thickness direction: Z direction) is smaller than dimensions in the width direction (X direction) and the depth direction (Y direction). The operation button 203 and the display panel 204 are provided on an upper surface 202a of the housing 202. However, the operation button 203 and the display panel 204 may be provided on a surface other than the upper surface 202a. The stepped portion 205 is constituted by an upper surface 205a formed so as to reduce a height of a part of the upper surface 202a of the housing 202, a side surface 205b which is a YZ plane connecting the upper surface 202a and the upper surface 205a to each other, and a side surface 205c which is an XZ plane connecting the upper surface 202a and the upper surface 205a to each other. The insertion opening 206 for the sensor 1 is formed in a lower part of the side surface 205c, and a receiving portion 207 (a female connector) which fits with an end portion on a side of one end 1a of the sensor 1 being inserted from the insertion opening 206 is provided inside the housing 202.

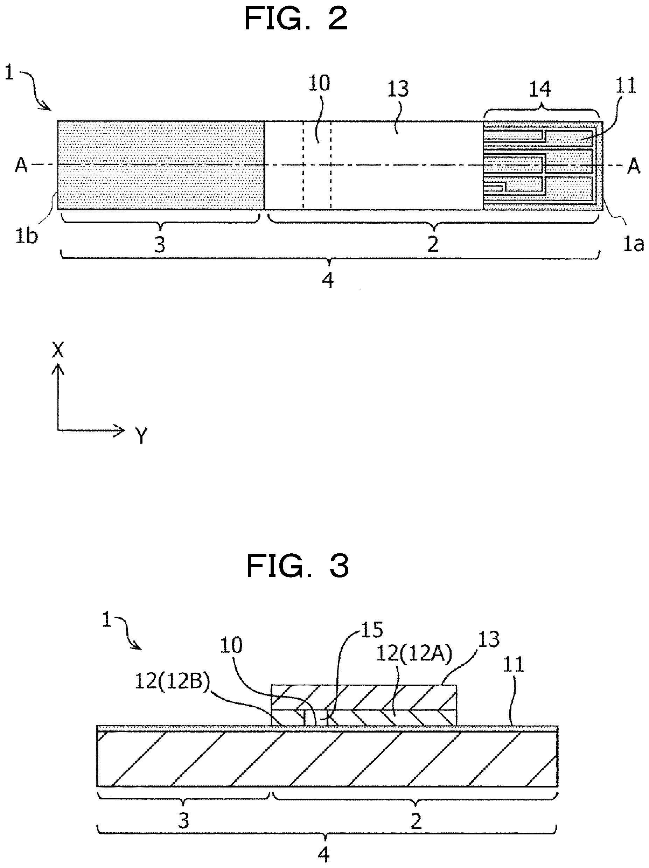

The sensor 1 is used to measure a target substance (a specific substance) contained in a sample (a specimen) . The sensor 1 is formed in a band-like plate shape having the one end 1a, another end 1b, an upper surface 1c, and a lower surface 1d, and has a longitudinal direction and a width direction. By inserting the one end 1a of the sensor 1 into the measurement apparatus by passing through the insertion opening 206, the sensor 1 is connected to the measurement apparatus 200. At this point, for example, the sensor 1 is relatively moved in a longitudinal direction (a Y direction) of the measurement apparatus 200 while bringing the lower surface 1d of the sensor 1 into contact with the upper surface 205a of the stepped portion 205 and, at the same time, bringing a left side surface of the sensor 1 into contact with the side surface 205b of the stepped portion 205. As a result, the end portion on the side of the one end 1a of the sensor 1 is inserted into the measurement apparatus 200 through the insertion opening 206 and fits with the receiving portion 207. In this manner, the end portion on the side of the one end 1a of the sensor 1 acts as a connecting portion 14 (a male connector, see Fig. 2) with the receiving portion 207. The connecting portion 14 corresponds to an inserting portion which is inserted into the measurement apparatus 200. The stepped portion 205 is used as a guide for guiding the side of the one end 1a (the connecting portion 14) of the sensor 1 to the insertion opening 206 of the measurement apparatus 200 in a preferable manner. The sensor 1 and the measurement apparatus 200 are electrically connected when the receiving portion 207 and the connecting portion 14 (see FIG. 2) are connected to each other.

In addition to the receiving portion 207 described above, a circuit board mounted with electronic parts necessary for prescribed operations (for example, applying voltage to a sample adhered to the sensor 1 and establishing a communication with the outside) of the measurement apparatus 200 is housed inside the housing 202. Examples of the electronic parts include a CPU, a RAM, and a ROM. In accordance with a program stored in the ROM, the CPU accepts an input made using the operation button 203 and performs a prescribed operation in accordance with the input. For example, in accordance with an input, the CPU causes the display panel 204 to display prescribed information (measurement conditions, a measurement result, and the like of the target substance). Alternatively, the CPU performs control of voltage application with respect to the sensor 1 (the sample), a measurement of the target substance based on a current (a response current) observed after the voltage application, and the like.

A user of the measurement system operates the measurement apparatus 200 using the operation button 203. A measurement result and the like in the measurement apparatus 200 are displayed on the display panel 204. The display panel 204 may be a touch panel. A person to be measured may operate the measurement apparatus 200 using the display panel 204.

### <Sensor>

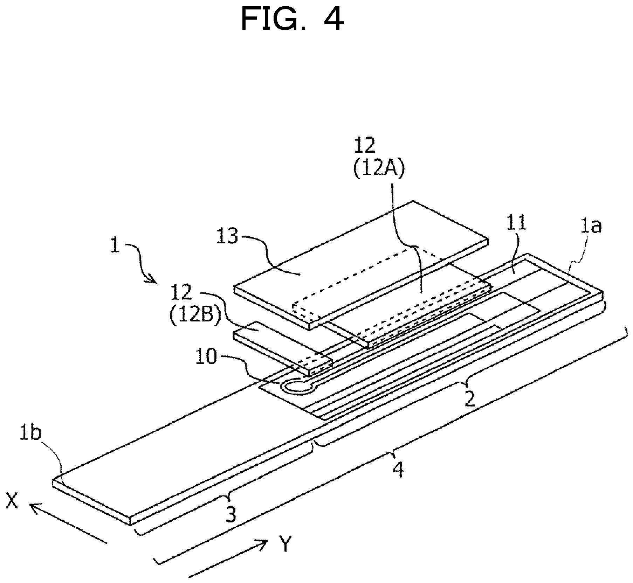

FIG. 2 is a plan view showing an example of the sensor (a sensor chip) 1 according to the embodiment. FIG. 3 is a sectional view showing an example of the sensor 1 according to the embodiment and represents a cross section taken along a dashed-dotted line A-A in FIG. 2. FIG. 4 is an exploded perspective view showing an example of the sensor 1 according to the embodiment.

The sensor 1 is an example of an "analysis tool" or a "test piece" and corresponds to a "sensor to which a sample is to be adhered". The sensor 1 includes a substrate 4 having a main body portion (a sensor portion) 2 and a holding portion (a handle portion) 3 which is connected to the main body portion 2. In FIGS. 2, 3, and 4, an arrangement direction of the main body portion 2 and the holding portion 3 is the Y direction and a direction perpendicular to the arrangement direction of the main body portion 2 and the holding portion 3 is the X direction. The arrangement direction of the main body portion 2 and the holding portion 3 aligns with a longitudinal direction of the main body portion 2. The direction perpendicular to the arrangement direction of the main body portion 2 and the holding portion 3 aligns with a transverse direction (a width direction) of the main body portion 2.

A spot application portion 10, a conductive layer 11, and a capillary member are provided on the main body portion 2. The capillary member includes a spacer 12 and a cover 13 which are provided on the conductive layer 11 formed in the main body portion 2 of the substrate 4.

The main body portion 2 has the connecting portion 14 which can be connected to a measurement apparatus that measures a target substance inside the sample. The sample can be spotted onto the spot application portion 10. In other words, the sample can be caused to adhere to the sensor 1. The spacer 12 and the cover 13 cover part of an upper surface of the main body portion 2 but do not cover the holding portion 3. In addition, the spacer 12 and the cover 13 cover a part of the conductive layer 11. The cover 13 is provided on the spacer 12. In the connecting portion 14, a part of the conductive layer 11 is exposed from the spacer 12 and the cover 13. The holding portion 3 and the spot application portion 10 are not electrically connected to each other.

More specifically, the sensor 1 includes the substrate 4 having the main body portion 2 and the holding portion 3 which is connected to the main body portion 2. The substrate 4 is formed in a band-like flat plate shape and has one surface (an upper surface) and a reverse-side surface (a lower surface) on an opposite side thereof. The spot application portion 10, the conductive layer 11, and part of the capillary member are provided on the upper surface of the main body portion 2. The upper surface and the lower surface are parallel planes as an example. The upper surface of the main body portion 2 corresponds to a plane on which a plurality of electrode pads is formed.

In the present embodiment, the capillary member includes spacers 12A and 12B and the cover 13. Each of the spacers 12A and 12B is a rectangular member having a dimension in a width direction which is approximately the same as a dimension of the substrate 4 in a width direction thereof, and the spacers 12A and 12B are arranged (bonded, laminated) in a state where a gap is provided in the X direction of the sensor 1 on the conductive layer 11 formed in the main body portion 2. The cover 13 is arranged in an overlapping manner on the spacers 12A and 12B so as to straddle the gap between the spacer 12A and the spacer 12B, and cover the spacers 12A and 12B.

An end portion of the main body portion 2 on a side of the one end 1a of the substrate 4 is not covered with the spacer 12A and the cover 13 and is in a state where the conductive layer 11 formed on the substrate 4 is exposed. The portion of the main body portion 2 where the conductive layer 11 is exposed is inserted into the insertion opening 206 of the sensor 1, and the portion of the main body portion 2 is used as the connecting portion 14 (a male connector) which fits with the receiving portion 207.

The conductive layer 11 which is exposed in the connecting portion 14 contacts with a connector pin (which corresponds to a conductive portion) arranged inside the receiving portion 207 and is used in order to electrically connect the sensor 1 and the measurement apparatus 200 to each other. The conductive layer 11 includes a prescribed number of (a plurality of) electrodes and a plurality of pads for connecting the respective electrodes to corresponding connector pins. An electrode and a corresponding pad are integrally formed such that there is no clear boundary between the electrode and the pad. Hereinafter, a combination of one electrode and a pad that corresponds to the electrode (that is integrally formed with the electrode) will be referred to as an "electrode pad". The conductive layer 11 is a group of a plurality of electrode pads.

A combination of two or more electrodes among the plurality of electrodes is used in order to measure the target substance or characteristics in the sample. While an example of the sample is a biological sample (also referred to as a living specimen), the sample is not limited to a biological sample. The biological sample is a liquid sample such as blood, interstitial fluid, or urine. The target substance in the sample is a glucose level (a blood sugar level), a lactate level (a level of tactic acid), or the like. In addition, characteristics in the sample are a hematocrit level, viscosity, a salt concentration, and the like of blood. In the present embodiment, the sensor 1 includes an electrode group used to measure a glucose level and an electrode group used to measure a hematocrit level (Hct level).

In the example shown in FIGS. 2 and 3, the spacer 12 and the cover 13 are not provided on the holding portion 3. However, the spacer 12 and the cover 13 may be configured so as to cover a part of the upper surface of the main body portion 2 as well as a part of or all of the upper surface of the holding portion 3. The holding portion 3 and the conductive layer 11 and the spot application portion 10 are not electrically connected to each other.

For example, the main body portion 2, the holding portion 3, the spacer 12, and the cover 13 are formed of an insulating base material such as a thermoplastic resin, a polyimide resin, an epoxy resin, glass, a ceramic, or paper. Thermoplastic resins include polyetherimide (PEI), polyethylene terephthalate (PET), and polyethylene (PE). The main body portion 2 and the holding portion 3 may be constituted by a single base material, constituted by a plurality of base materials of a same type, or constituted by base materials of two or more types. The main body portion 2 and the holding portion 3 may be constituted by the same base material. The main body portion 2 and the holding portion 3 may be constituted by different base materials.

The sensor 1 may be a rectangle, a rectangle with rounded corners, or an ellipse when viewed from a normal direction (in a plan view) of the upper surface of the main body portion 2. In a plan view, the main body portion 2 may be a rectangle and the holding portion 3 may be a rectangle with rounded corners. In a plan view, the main body portion 2 may be a rectangle with rounded corners and the holding portion 3 may be a rectangle. The conductive layer 11 is formed on the upper surface of the main body portion 2 of the substrate 4. The conductive layer 11 is formed by a conductive substance (a conductor). For example, the conductive layer 11 is formed using a metallic material such as gold (Au), silver (Ag), platinum (Pt), palladium (Pd), or ruthenium (Ru), or a carbon material such as carbon. Each of the electrode pads constituting the conductive layer 11 can be formed as a metal layer having a desired thickness by, for example, firm formation by physical vapor deposition (PVD, such as sputtering) or chemical vapor deposition (CVD) of the metallic material. Alternatively, each of the electrode pads may be formed by printing ink containing a carbon material or metal particles on the substrate 4 by screen printing. The conductive layer 11 is formed with an approximately uniform thickness (a dimension in the Z direction).

The spot application portion 10 is formed by the spacers 12A and 12B and the cover 13. A gap (a notch) is formed between the spacer 12A and the spacer 12B in a direction perpendicular to the arrangement direction of the main body portion 2 and the holding portion 3. In the gap portion, a part of the conductive layer 11 formed on an upper surface of the substrate 4 is exposed, and a space defined by the exposed upper surface (the conductive layer 11) of the substrate 4, side surfaces of the spacers 12A and 12B, and an inner surface of the cover 13 forms the spot application portion 10. Each of end portions of the space in the width direction of the sensor 1 (the X direction) is opened. In other words, end portions of the space on both side surfaces of the sensor 1 are open ends. When a sample is brought into contact with (spotted onto) one of the two openings, the sample is sucked into the space by capillary action. At this point, the other opening acts as an air hole through which air dislodged by the sample sucked into the space is discharged. In this manner, the space constitutes a flow path (a capillary) 15 of the sample. The open ends of the flow path 15 are an example of a feed port of the sample.

A reagent 17 to react with the sample can be provided inside the flow path 15. The reagent 17 can be arranged, placed, ,mounted, or immobilized on the electrodes exposed inside the flow path 15. The reagent 17 contains an enzyme and a substrate. In other words, the reagent 17 contains a salt. In some cases, the reagent 17 may contain a mediator. The enzyme is selected as appropriate in accordance with a type of the sample and the target substance. When the target substance is glucose in blood or interstitial fluid, glucose oxidase (GOD) or glucose dehydrogenase (GDH) is applied. Examples of the mediator include a ferricyanide, a ruthenium complex, p-benzoquinone, a p-benzoquinone derivative, phenazine methosulfate, methylene blue, ferrocene, and a ferrocene derivative. Among these substances, a ferricyanide or a ruthenium complex is favorable and potassium ferricyanide or a ruthenium compound [Ru(NH₃)₆]Cl₃ is more favorable.

### <Connecting Portion of Sensor and Receiving Portion of Measurement Apparatus>

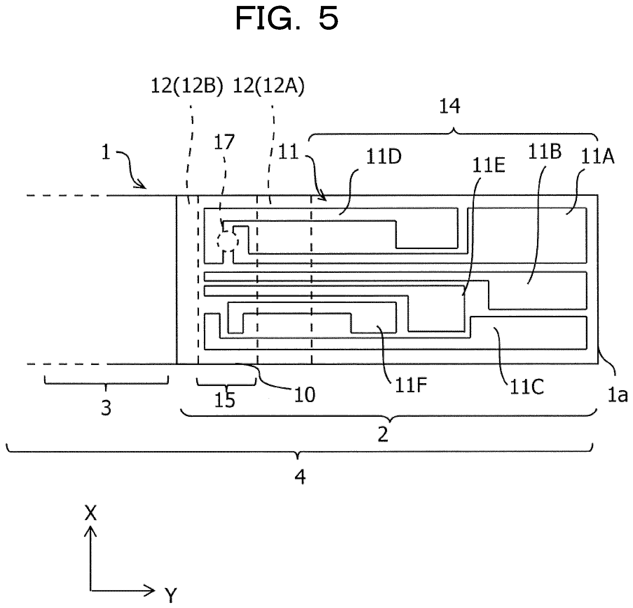

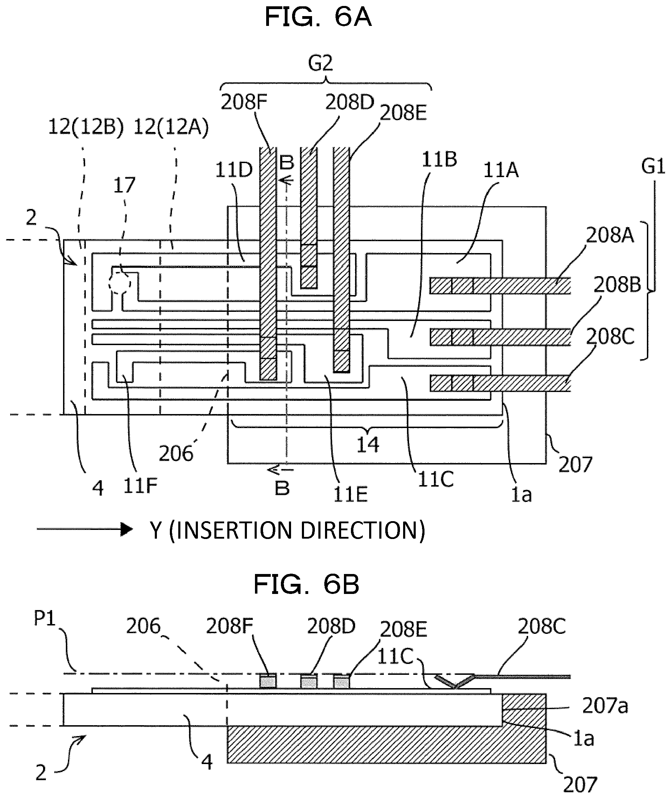

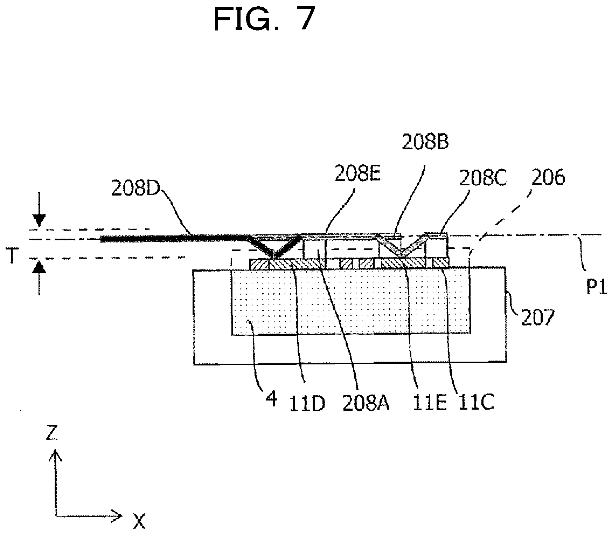

Next, the connecting portion 14 of the sensor 1 and the receiving portion 207 of the measurement apparatus 200 will be described in detail. FIG. 5 is a plan view showing an enlargement of the main body portion 2 of the sensor 1. FIG. 6A is a plan view schematically showing a state where the connecting portion 14 is fitted with the receiving portion 207, and FIG. 6B is a side view schematically showing a left side surface (a lower side in FIG. 6A) of the connecting portion 14 and the receiving portion 207 shown in FIG. 6A. FIG. 7 shows a cross section taken along a dashed-dotted line B-B in FIG. 6A. However, for the sake of brevity, shapes of the electrode pads of the conductive layer 11 illustrated in FIGS. 5, 6A, 6B, and 7 differ from the shapes illustrated in FIGS. 2 to 4.

In the example shown in FIG. 5, the conductive layer 11 formed on the upper surface (a plane) of the main body portion 2 includes an electrode pad 11A, an electrode pad 11B, an electrode pad 11C, an electrode pad 11D, an electrode pad 11E, and an electrode pad 11F as an example of the plurality of electrode pads. The electrode pad 11A, the electrode pad 11B, the electrode pad 11C, the electrode pad 11D, the electrode pad 11E, and the electrode pad 11F have a similar thickness and surfaces thereof are on a plane that is parallel to the upper surface of the main body portion 2. Among the electrode pad 11A, the electrode pad 11B, the electrode pad 11C, the electrode pad 11D, the electrode pad 11E, and the electrode pad 11F, portions exposed in the sample flow path 15 formed between the spacer 12A and the spacer 12B are used as electrodes. In addition, among the electrode pad 11A, the electrode pad 11B, the electrode pad 11C, the electrode pad 11D, the electrode pad 11E, and the electrode pad 11F, portions exposed in the connecting portion 14 (not covered by the spacer 12A) are used as pads to be used to establish an electric connection to the measurement apparatus 200.

As shown in FIG. 1, a position of the measurement apparatus 200 is fixed, the one end 1a of the sensor 1 is oriented toward the measurement apparatus 200, and the sensor 1 is relatively moved in a depth direction (the Y direction) of the measurement apparatus 200 along the upper surface 205a of the stepped portion 205. Accordingly, as shown in FIGS. 6A, 6B, and 7, the connecting portion 14 of the sensor 1 passes through the insertion opening 206 and fits with the receiving portion 207. The receiving portion 207 includes a locking surface 207a provided in a height direction (the Z direction) of the measurement apparatus 200, and the connecting portion 14 of the sensor 1 is inserted into the measurement apparatus 200 to a position (an example of the prescribed position) where the one end 1a of the sensor 1 abuts with the locking surface 207a to create a state where the connecting portion 14 is fitted with the receiving portion 207. Such a state represents an example of an inserted state (indicating a stage an inserting portion is inserted within a measurement apparatus) of the sensor 1 with respect to the measurement apparatus 200. Alternatively, a configuration may be adopted in which a side surface of the spacer 12A or the cover 13 on the side of the one end 1a of the sensor 1 abuts the side surface 205c (FIG. 1) so as to restrict an insertion depth of the sensor 1.

The receiving portion 207 has a plurality of connector pins 208A to 208F (which corresponds to the plurality of conductive portions) arranged inside the measurement apparatus 200. When the plurality of connector pins 208A to 208F are not distinguished from one another, the plurality of connector pins will be described as the connector pin 208. Each connector pin 208 is formed by a conductor made of a metal or carbon and, by coming into contact with a surface of a corresponding electrode pad, the connector pin 208 electrically connects a corresponding electrode to the measurement apparatus 200. In other words, "coming into contact" means creating a state where an electrode pad and a connector pin can be electrically connected to each other.

In the present embodiment, the measurement apparatus 200 includes a first connector pin group G1 (which corresponds to a first conductive portion group) which extends in an insertion direction of the sensor 1 into the measurement apparatus 200 and a second connector pin group G2 (which corresponds to a second conductive portion group) which extends in a direction intersecting the insertion direction. In other words, each of the plurality of connector pins 208 belongs to one of the first connector pin group G1 and the second connector pin group G2. An insertion direction of the sensor 1 indicates a direction of the sensor 1 when the connecting portion 14 (the inserting portion) is inserted within the measuring apparatus 200 passing through the insertion opening 206 (Y direction in a sample illustrated in Fig. 1). Each of the plurality of connecter pins 208 (conductive portions) has one end (base end) and other end (tip), the one end is supported in the measuring apparatus 200, and the other end is contactable the plurality of electrode pads formed on the plane (upper surface) of the inserting portion (connection portion 14) in the inserted state of the sensor 1.

In the present embodiment, the first connector pin group G1 includes the connector pin 208A, the connector pin 208B, and the connector pin 208C corresponding to the electrode pads 11A, 11B, and 11C. The connector pin 208A contacts with an upper surface of the electrode pad 11A in the inserted state of the sensor 1. The connector pin 208B contacts with an upper surface of the electrode pad 11B in the inserted state of the sensor 1. The connector pin 208C contacts with an upper surface of the electrode pad 11C in the inserted state of the sensor 1. In this manner, the first connector pin group G1 (the first conductive portion group) contacts with the electrode pads 11A, 11B, and 11C which correspond to the first connector pin group G1 among the plurality of electrode pads.

The second connector pin group G2 includes the connector pin 208D, the connector pin 208E, and the connector pin 208F corresponding to the electrode pads 11D, 11E, and 11F. The connector pin 208D contacts with an upper surface of the electrode pad 11D in the inserted state of the sensor 1. The connector pin 208E contacts with an upper surface of the electrode pad 11E in the inserted state of the sensor 1. The connector pin 208F contacts with an upper surface of the electrode pad 11F in the inserted state of the sensor 1. In this manner, the second connector pin group G2 contacts with electrode pads that do not contact with the first connector pin group G1 (electrode pads other than the electrode pads contacting the first connecter pin group G1) in the inserted state of the sensor 1 among the plurality of electrode pads 11A to 11F. In other words, the second connector pin group G2 (the second conductive portion group) contacts with the electrode pads 11D, 11E, and 11F other than the electrode pads 11A, 11B, and 11C which correspond to the first connector pin group G1 among the plurality of electrode pads. In this manner, when a side of the insertion opening 206 of the receiving portion 207 is considered a near side and a side of the locking surface 207a is considered a far side in the insertion direction of the sensor 1 (the Y direction in Fig. 1), the first connector pin group G1 and the electrode pads 11A, 11B, and 11C corresponding thereto are arranged on the far side in the insertion direction than the second connector pin group G2 and the electrode pads 11D, 11E, and 11F corresponding thereto. In other words, the second connector pin group G2 and the electrode pads 11D, 11E, and 11F corresponding thereto are arranged on the near side in the inserting portion than the first connector pin group G1 and the electrode pads 11A, 11B, and 11C corresponding thereto.

The connector pin 208A, the connector pin 208B, and the connector pin 208C extend in the Y direction (the insertion direction of the sensor 1) on an XY plane of the measurement apparatus 200. By comparison, the connector pin 208D, the connector pin 208E, and the connector pin 208F extend in the X direction that is perpendicular to the Y direction (an example of a direction intersecting the insertion direction of the sensor) on the XY plane of the measurement apparatus 200. Hypothetically, when the plurality of connector pins 208A to 208F are arranged in the width direction (the X direction), a size of the sensor 1 in the width direction also increases. By extending the connector pin 208D, the connector pin 208E, and the connector pin 208F in a direction perpendicular to the insertion direction as in the present embodiment, a reduction in the size of the sensor 1 in the width direction is realized.

It should be noted that an angle at which the connector pins belonging to the second connector pin group G2 intersect with the insertion direction need not be a right angle. In addition, angles at which the respective connector pins belonging to the second connector pin group G2 intersect with the insertion direction may differ from each other. Furthermore, an angle at which a direction of each connector pin 208 belonging to the first connector pin group G1 intersects with the insertion direction of the sensor 1 may be set to a first range (for example, ±5 to 10 degrees), and an angle at which a direction of each connector pin 208 belonging to the second connector pin group G2 intersects with the insertion direction of the sensor 1 may be defined as an angle greater than the first range.

Each connector pin 208 is formed in a band shape having a prescribed thickness, a tip portion thereof is bent in a V-shape or a U-shape, and a bottom portion of the V-shape or the U-shape contacts with a corresponding pad. Accordingly, a surface of a pad is prevented from being damaged by the contact between the connector pin 208 and the pad. In addition, each connector pin 208 has elasticity and the tip portion is in a state of being tensioned downward, and each connector pin 208 is configured such that the tip portion presses down on an upper surface (a surface) of a corresponding pad during insertion of the connecting portion 14. However, the shape of the tip portion is optional and the connector pin 208 may have a simple band shape, strip shape, or rod shape.

Insofar as each of the plurality of connector pins 208 is able to form a preferable contact state with a corresponding electrode pad, there is no limit to a height of a part (e.g. the surface) of the connector pin 208 itself is provided. However, from the perspective of forming a thin measurement apparatus 200 (reducing the size of the measurement apparatus), for example, the plurality of connector pins 208 are provided as follows. Specifically, in the inserted state, each of the plurality of connector pins 208 has a farthest portion apart from the plane (the upper surface of the connection portion 14) in the normal direction of the plane. A distance in the normal direction between the plane and the farthest portion is set so that the distance is shorter than a given distance. In the present embodiment, a portion (a base end portion: also referred to as an arm portion) on an opposite side to the tip portion of the plurality of connector pins 208 is the most distal portion furthest from the upper surface of the main body portion 2, and the arm portion is arranged on a same plane (a virtual plane P1 indicated by a dashed-dotted line) which is approximately parallel to the plane of the sensor 1 (the upper surface of the main body portion 2 (including connection portion 14)) on which the conductive layer 11 is formed. In other words, in the inserted state of the sensor 1, the plurality of connector pins 208 are provided such that a distance (a height) between the upper surface 1c of the sensor 1 (the one surface of the main body portion 2 on which the conductive layer is formed) of the connecting portion 14 (the inserting portion) and each of the plurality of connector pins 208 in a thickness direction of the measurement apparatus 200 (the Z direction: which is also the normal direction of the upper surface of the main body portion 2 of the sensor 1 in an inserted state) is within a prescribed range T (equal to or shorter than T). In this manner, by restricting heights of the connector pins 208 by varying directions in which the connector pins 208 extend on a same plane, even when a large number of connector pins 208 are provided, an increase in the size of the measurement apparatus 200 in the thickness direction (the Z direction) is suppressed. In the present embodiment, in the inserted state of the sensor 1, a surface of the conductive layer 11 (the plurality of electrode pads) is on a plane parallel to the upper surface of the main body portion 2. In other words, in the inserted state, a level of surface of each of the plurality of electrode pads in the horizontal direction of the plane (the upper surface of the connection portion 14) is the same. In addition, the plurality of connector pins 208 are provided such that a distance from the surface of each of the plurality of electrode pads to a most distal portion (the arm portion) of each of the plurality of connector pins 208 in the normal direction relative to the surface of the plurality of electrode pads (the Z direction of the measurement apparatus 200) is within 1.5 times a thickness of each of the plurality of connector pins 208. Note that, a configuration may apply that in the inserted state of the sensor 1, each of the plurality of contact pads 208 may be arranged on a plane that is parallel to the upper surface of the connection portion (the plane included in the inserting portion).

While six connector pins 208 which correspond to six electrode pads are provided in the present embodiment, the number of connector pins 208 can be changed as appropriate in accordance with the number of electrode pads. In addition, the number of connector pins 208 respectively belonging to the first and second connector pin groups G1 and G2 can be changed as appropriate in accordance with a layout of components inside the measurement apparatus 200.

### <Measurement of target substance and the like>

Next, an example of a measurement using the sensor 1 and the measurement apparatus 200 will be described. As an example of measurement, a case where a glucose level and an Hct level in blood are measured using the sensor 1 will be described. Blood is an example of a sample, the glucose level is an example of a target substance in the sample, and the Hct level is an example of characteristics.

### <Configuration of Sensor>

In the present embodiment, among the electrode pads 11A to 11F (FIG. 5) included in the sensor 1, electrode portions (portions exposed in the flow path 15) of the electrode pad 11D and the electrode pad 11A are used as an electrode pair (a working electrode and a counter electrode) for measuring a glucose level. In addition, electrode portions (portions exposed in the flow path 15) of the electrode pad 11C and the electrode pad 11F are used as an electrode pair for measuring an Hct level. The electrode pad 11B and the electrode pad 11E are to be used for other applications.

### <Configuration of Measurement Apparatus>

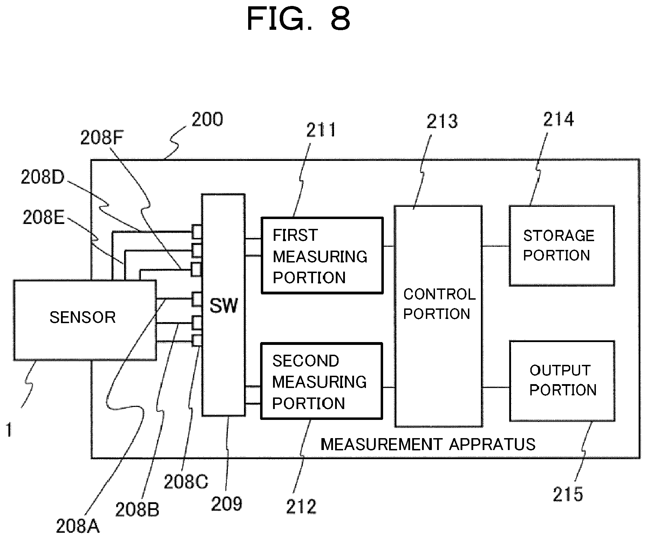

FIG. 8 shows a configuration example of the measurement apparatus 200. The measurement apparatus 200 includes a switch (SW) 209, a first measuring portion 211, a second measuring portion 212, a control portion 213, a storage portion 214, and an output portion 215.

The switch 209 is connected to the connector pins 208A to 208F described above. The switch 209 is a switch for switching between states of electrical connection and disconnection to each electrode of the sensor 1 in the inserted state. For example, when measuring a glucose level, the control portion 213 controls the switch 209 to switch the electrode pair (the electrode pads 11A and 11D) for glucose level measurement to an electrically connected state to the measurement apparatus 200 and to switch the electrode pads other than the electrode pads 11A and 11D to a disconnected state. The first measuring portion 211 applies prescribed DC voltage (a first signal) to the electrode pads 11A and 11D and measures a first electrical response to the first signal.

When measuring an Hct level, the control portion 213 controls the switch 209 to switch the electrode pair (the electrode pads 11B and 11E) for measuring an Hct level to an electrically connected state to the measurement apparatus 200 and to switch the electrode pads other than the electrode pads 11B and 11E to a disconnected state. The second measuring portion 212 applies prescribed DC voltage (a second signal) to the electrode pads 11B and 11E and measures a second electrical response value after a lapse of a prescribed period of time from start of application (at a time point where the application has continued for a prescribed period of time after the start of the application).

The storage portion 214 includes a main storage apparatus such as a RAM or a ROM and an auxiliary storage apparatus such as a hard disk. The storage portion 214 stores a program to be executed by the control portion 213, data used by the control portion 213 when executing the program, and the like. The control portion 213 includes a processor (such as a CPU) which executes the program stored in the storage portion 214. The output portion 215 includes an output apparatus such as a printer or the display panel 204 and a communication device such as a signal connector or a communication interface.

The storage portion 214 stores calibration curve data (a calibration curve table) for obtaining a glucose concentration from the first electrical response value measured by the first measuring portion 211. The control portion 213 calculates a glucose level by converting the first electrical response value into a glucose level using the calibration curve table. The glucose level is stored in the storage portion 214 and/or output (displayed or the like) from the output portion 215.

In addition, the storage portion 214 stores calibration curve data (a calibration curve table) for obtaining an Hct level from the second electrical response value measured by the second measuring portion 212. The control portion 213 calculates an Hct level by converting the second electrical response value into an Hct level using the calibration curve table. The Hct level can be stored in the storage portion 214 and/or output (displayed or the like) from the output portion 215.

Furthermore, the control portion 213 performs a process of correcting a glucose level using the second electrical response value obtained from the second measuring portion 212 or an Hct level converted from the second electrical response value. For example, the storage portion 214 stores calibration curve data (a calibration curve table) indicating a correspondence relationship between the second electrical response value and a correction amount. The control portion 213 obtains a correction amount corresponding to the second electrical response value using the calibration curve table, performs a process of correcting a glucose level, and calculates the corrected glucose level. The corrected glucose level can be stored in the storage portion 214 and/or output (displayed, transmitted, or the like) from the output portion 215. It should be noted that, when the second electrical response value is used to correct a glucose level, a configuration for converting the second electrical response value into an Hct value need not necessarily be provided.

### <<Operation Example>>

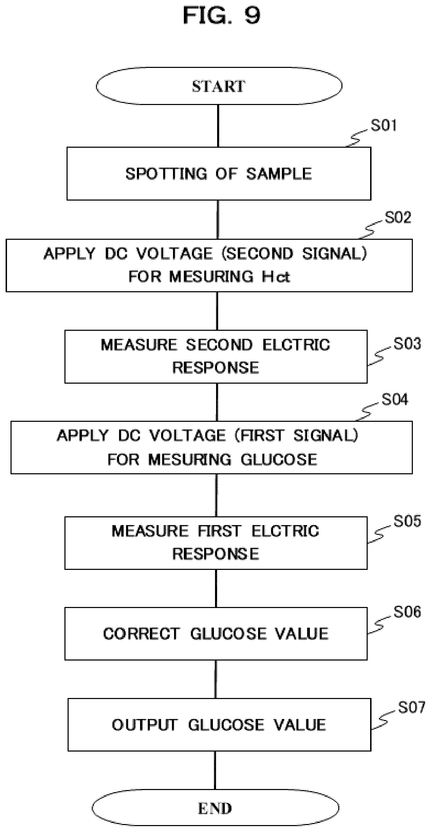

FIG. 9 is a flow chart showing an operation example of the measurement apparatus 200. In S01 shown in FIG. 9, spotting of a sample is performed. Specifically, the connecting portion 14 of the sensor 1 (a biosensor) is inserted from the insertion opening 206 and fitted with the receiving portion 207. In other words, the sensor 1 is physically connected to the measurement apparatus 200. Next, blood (the sample) collected from an examinee is brought into contact with (spotted onto) the spot application portion 10 of the sensor 1 (an open end of the flow path 15). Accordingly, the blood is sucked into the flow path 15 by capillary force and fills the inside of the flow path 15.

Prior to spotting, the control portion 213 applies voltage to a prescribed electrode, measures the applied voltage, detects a change in the voltage caused by the blood and the electrode coming into contact with each other due to the spotting, and detects that the blood has been introduced into the flow path 15. Accordingly, the control portion 213 starts measuring an Hct value (S02) .

In S02, the control portion 213 controls the switch 209 to switch the electrode pair (the electrode pads 11B and 11E) for measuring an Hct level to an electrically connected state to the measurement apparatus 200 and controls the second measuring portion 212 to apply a second signal to the blood. In other words, the control portion 213 issues an instruction to the second measuring portion 212 and causes DC voltage that corresponds to the second signal to be applied to the electrode pair for Hct measurement.

The second measuring portion 212 waits for the application of the DC voltage corresponding to the second signal to continue for a prescribed period of time from a start of the application, and measures a second electrical response of the blood at a time point where the application has continued for the prescribed period of time (S03) . For example, the second measuring portion 212 measures a response signal with respect to the DC voltage, subjects the response signal to A/D conversion, and sends the converted response signal to the control portion 213.

Once a second electrical response of blood to the second signal is acquired, the control portion 213 starts a measurement of a glucose level. Specifically, the control portion 213 applies a first signal to the blood (S04) . More specifically, the control portion 213 instructs the first measuring portion 211 to apply, as the first signal, DC voltage that is lower than the second signal to the electrode pair (the electrode pads 11A and 11D) for glucose measurement. The first measuring portion 211 applies the DC voltage in accordance with the instruction.

The first measuring portion 211 measures a first electrical response of the blood to the first signal (S05). For example, the first measuring portion 211 measures a response current with respect to the DC voltage applied as the first signal. The first measuring portion 211 subjects the response current to A/D conversion and sends the converted response current to the control portion 213.

The control portion 213 operates as a correcting portion and performs a correction process of a glucose level (S06) . In other words, the control portion 213 calculates a value of a correction object component (a glucose level) contained in blood using a value of the first electrical response (the response current) acquired in S05 and the calibration curve data (the calibration curve table). In addition, the control portion 213 corrects the calculated glucose level using the second electrical response value acquired in S03 (or an Hct level corresponding to the second electrical response value).

The control portion 213 outputs the corrected glucose level (S07). Specifically, the control portion 213 stores the glucose level corrected in S06 in the storage portion 214 and displays the glucose level on the output portion 215 (a display). Using the output portion 215, the control portion 213 can also transmit the glucose level to another apparatus via a wired or wireless network.

### <Advantageous Effect of Embodiment>

The measurement apparatus 200 according to the first embodiment has connector pins belonging to the first connector pin group G1 and connector pins belonging to the second connector pin group G2 as the plurality of connector pins 208. to be connected to the plurality of electrode pads included in the sensor 1. Therefore, an increase in the size of the sensor 1 in the width direction can be avoided and, at the same time, an increase in the size of the measurement apparatus 200 in the height direction (thickness) can be avoided. As a result, the sensor 1 and the measurement apparatus 200 can be downsized and management of storage, portability, and the like can be readily performed.

### <First Modification>

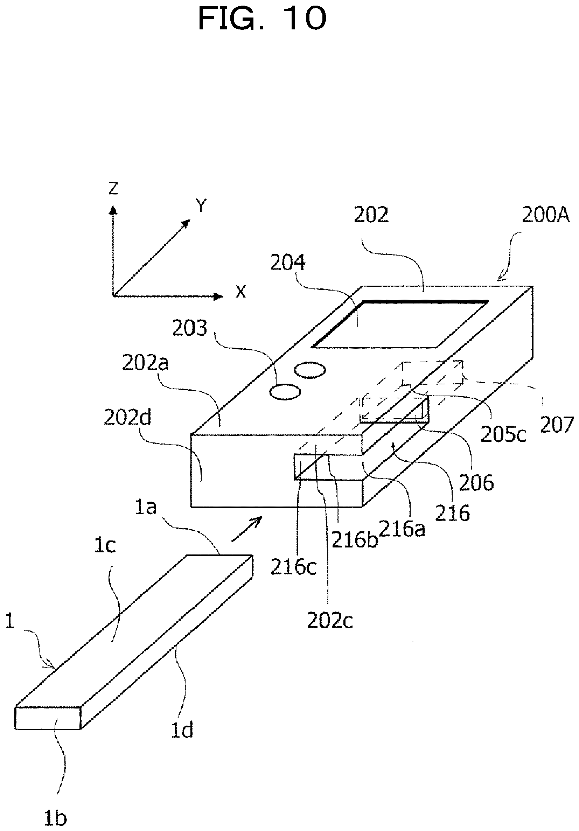

FIG. 1 shows an example in which the measurement apparatus 200 has the stepped portion 205 as a guide. However, the guide may be a groove which is provided on a side surface of the housing 202 and which has a depth comparable to the size of the sensor 1 in the width direction. FIG. 10 shows a modification of the measurement apparatus 200. In FIG. 10, in a measurement apparatus 200A according to the modification, a groove 216 which is recessed in the width direction (the X direction) of the measurement apparatus is formed on a near side of the insertion opening 206. A depth (a depth in the X direction) of the groove 216 is formed slightly greater than the dimension of the sensor 1 in the width direction, and the groove 216 has an upper surface 216a to oppose the lower surface 1d of the sensor 1 and a lower surface 216b to oppose the upper surface 1c of the sensor 1. When connecting the sensor 1 to the measurement apparatus 200A, the one end 1a of the sensor 1 can be readily guided to the insertion opening 206 by sliding the sensor 1 in the Y direction while bringing the left side surface of the sensor 1 into contact with a bottom surface 216c of the groove 216. It should be added that a portion where the lower surface 216b of the groove 216 is not provided need not be flush with the near-side end surface 202d and may be formed on a far side of the end surface 202d.

### <Second Modification>

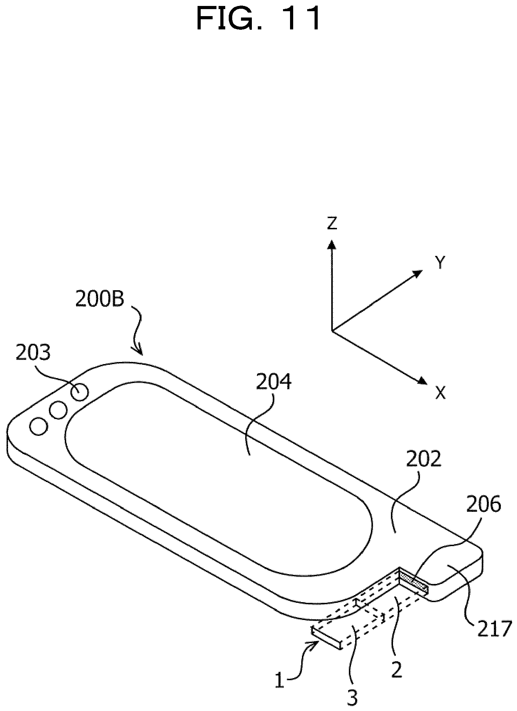

Alternatively, the measurement apparatus may be configured as shown in FIG. 11. FIG. 11 shows a second modification of the measurement apparatus. A measurement apparatus 200B shown in FIG. 11 includes a protruded portion 217 which protrudes sideways from an end portion of the housing 202 in a longitudinal direction. The insertion opening 206 of the sensor 1 is provided on a side surface of the protruded portion 217, and a user of the measurement apparatus 200B inserts the tip of the main body portion 2 into the insertion opening 206 while holding the holding portion 3 (to be described later) of the sensor 1. When the tip of the main body portion 2 is inserted into the insertion opening 206, the protruded portion 217, the insertion opening 206, the main body portion 2, and the holding portion 3 are arranged in a single line. In addition, when the tip of the main body portion 2 is inserted into the insertion opening 206, the main body portion 2 and the housing 202 are arranged adjacent to each other in a direction perpendicular to the arrangement direction of the protruded portion 217, the insertion opening 206, the main body portion 2, and the holding portion 3. Therefore, when a sample is spotted onto the spot application portion 10 from a side of the side surface of the main body portion 2 in a state where the tip of the main body portion 2 is inserted into the insertion opening 206, the sample is caused to approach the main body portion 2 in a direction perpendicular to the arrangement direction of the protruded portion 217, the insertion opening 206, the main body portion 2, and the holding portion 3. In this case, since a hand or a finger of a person to be measured does not contact with the housing 202 and the protruded portion 217, the sample can be more readily spotted onto the spot application portion 10 from the side of the side surface of the main body portion 2.

The protruded portion 217 protrudes in a direction perpendicular to the arrangement direction of the protruded portion 217, the insertion opening 206, the main body portion 2, and the holding portion 3. In order to make it easier for the sample to be spotted onto the spot application portion 10 from the side of the side surface of the main body portion 2, favorably, a length of the protruded portion 217 in the direction perpendicular to the arrangement direction of the protruded portion 217, the insertion opening 206, the main body portion 2, and the holding portion 3 is slightly greater than a length (W1) of the sensor 1 in a direction perpendicular to the arrangement direction of the main body portion 2 and the holding portion 3. For example, the length of the protruded portion 217 in the direction perpendicular to the arrangement direction of the protruded portion 217, the insertion opening 206, the main body portion 2, and the holding portion 3 may be at least equal to and not more than double the length (W1) of the sensor 1 in the direction perpendicular to the arrangement direction of the main body portion 2 and the holding portion 3.

### Second Embodiment

A measurement system according to a second embodiment will be described. Since the second embodiment shares the configuration of the first embodiment, differences will be mainly described below and descriptions of common points will be omitted. As a measurement apparatus according to the second embodiment, the measurement apparatuses 200, 200A, and 200B described in the first embodiment can be applied. However, the second embodiment differs from the first embodiment in that components and processes related to specifications of the sensor 1 have been added.

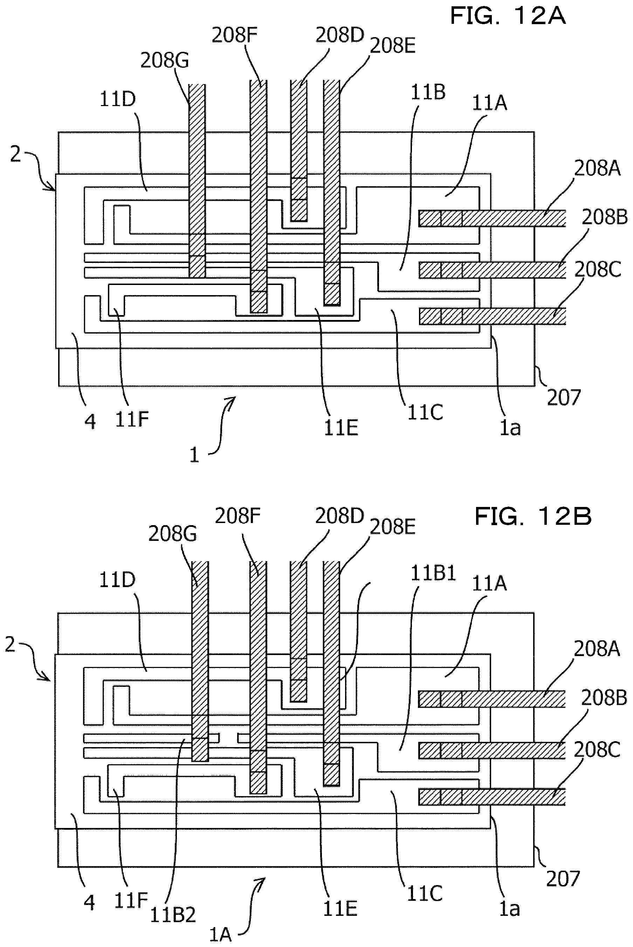

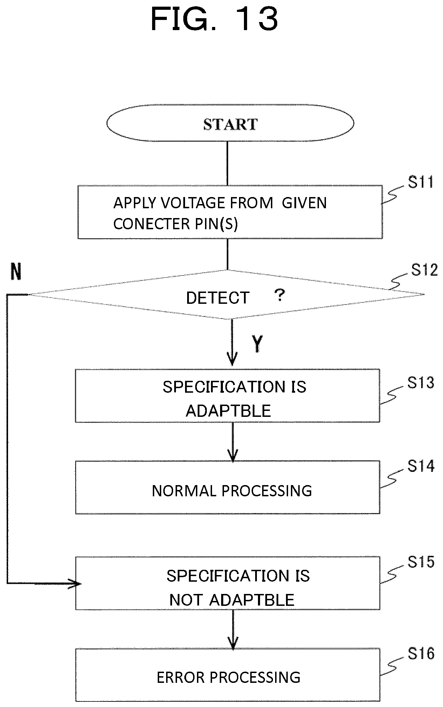

FIGS. 12A and 12B show configuration examples of a sensor and a measurement apparatus according to the second embodiment. FIG. 13 is a flow chart showing an example of processes related to a determination of specifications of a sensor. As shown in FIG. 12A, the measurement apparatus 200 according to the second embodiment further includes a connector pin 208G belonging to the second connector pin group G2. Meanwhile, as a sensor, the sensor 1 described in the first embodiment and a sensor 1A with specifications that differ from those of the sensor 1 are prepared.

For example, while the sensor 1 and the sensor 1A share a same configuration, the sensor 1 and the sensor 1A have different points of destination (regions in which the sensors are to be circulated) . For example, the sensor 1 is intended to be used in Asia and the sensor 1A is intended to be used in Europe. It is assumed that the sensor 1 for Asia cannot be used in Europe and vice versa.

As shown in FIG. 12B, in the sensor 1A, the electrode pad 11B which is a single electrode pad in the sensor 1 has been divided into two electrode pads 11B1 and 11B2 which are respectively insulated.

The connector pin 208G contacts with the electrode pad 11B as shown in FIG. 12A in the inserted state of the sensor 1. In contrast, the connector pin 208G contacts with the electrode pad 11B2 as shown in FIG. 12B in the inserted state of the sensor 1A. The connector pin 208B contacts with the electrode pad 11B1.

The process shown in FIG. 13 starts when, for example, a sensor (any of the sensor 1 and the sensor 1A) is inserted to the measurement apparatus 200. The control portion 213 applies voltage between prescribed connector pins which are the connector pin 208B and the connector pin 208G in the present embodiment (S11), and determines whether a change occurs in a current (S12).

When the inserted sensor is the sensor 1, since the connector pin 208B and the connector pin 208G constitute a closed circuit via the electrode pad 11B, a change in a current value due to the voltage application is detected. In this case, the process advances to S13. On the other hand, when the inserted sensor is the sensor 1A, since the connector pin 208B and the connector pin 208G constitute an open circuit, a current change does not occur. In this case, the process advances to S15.

When the process advances to S13, the control portion 213 determines that the inserted sensor is compatible (specifications are normal) and enters a state where an ordinary process (the process described with reference to FIG. 9) is to be performed (S14). In contrast, when the process advances to S15, the control portion 213 determines that the inserted sensor is incompatible (specifications are not normal) and performs a prescribed error process (S16) . For example, the control portion 213 stops the operation of the measurement apparatus 200 and causes a message to the effect that the sensor is unusable to be displayed on the display panel 204 included in the output portion 215. It should be noted that a combination of detection/ non-detection of a current change and a determination of compatible/ incompatible may be reversed.

With the exception of the above, the configuration of the second embodiment is similar to that of the first embodiment. According to the second embodiment, the control portion 213 operates as a determining portion by detecting a change in a current. Specifically, as a determining portion, the control portion 213 determines whether the connector pin 208B (which corresponds to a first conductive portion) in the first connector pin group G1 and the connector pin 208G (which corresponds to a second conductive portion) in the second connector pin group G2 are in contact with a same electrode pad or in contact with two different electrode pads among the plurality of electrode pads.

Therefore, with the measurement apparatus 200 according to the second embodiment, when a sensor is configured so as to detect a difference in specifications such as a point of destination, the connector pin 208G for specification determination is configured as a connector pin belonging to the second connector pin group G2. According to the second embodiment, an increase in a size of a measurement apparatus including a mechanism for determining specifications of a sensor and an increase in a size of the sensor can be suppressed.

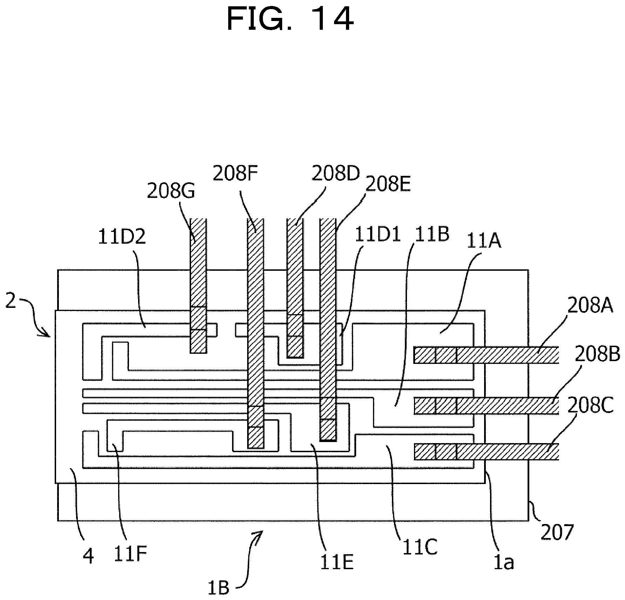

Alternatively, both connector pins to be used for the voltage application (determination of point of destination) described above may be connector pins belonging to the second connector pin group G2. FIG. 14 shows a modification of the second embodiment. In FIG. 14, in a sensor 1B, the electrode pad 11D has been divided into two electrode pads 11D1 and 11D2 which are respectively insulated. In an inserted state of the sensor 1B into the measurement apparatus, the electrode pad 11D1 contacts with the connector pin 208D belonging to the second connector pin group G2, and the electrode pad 11D2 contacts with the connector pin 208G belonging to the second connector pin group G2. In this case, the process shown in FIG. 13 is performed with respect to both the connector pin 208D and the connector pin 208G. In this manner, the control portion 213 may be configured so as to operate as a determining portion which determines, using two connector pins 208B and 208G which belong to the second connector pin group G2, whether the connector pins 208B and 208G are in contact with a same electrode pad 11D or in contact with two different electrode pads 11D1 and 11D2 among the plurality of electrode pads.

### Third Embodiment

A measurement system according to a third embodiment will be described. Since the third embodiment shares the configuration of the first embodiment, differences will be mainly described below and descriptions of common points will be omitted. As a measurement apparatus according to the third embodiment, the measurement apparatuses 200, 200A, and 200B described in the first embodiment can be applied. However, the third embodiment differs from the first embodiment in a configuration of connector pins belonging to the second connector pin group G2.

In the first embodiment, each of the connector pins 208D, 208E, and 208F which belong to the second connector pin group G2 are arranged so as to rub against a plurality of electrode pads (the electrode pads 11A to 11F) inside the receiving portion 207 during insertion of the sensor 1. In this case, "rub against" means that objects contact with each other and move while creating friction. Subsequently, when the one end 1a of the sensor 1 reaches a position (a prescribed position) where the one end 1a abuts with the locking surface 207a, each of the connector pins 208D, 208E, and 208F c contacts with corresponding electrode pads 11D, 11E, and 11F and becomes electrically connected (FIG. 6A).

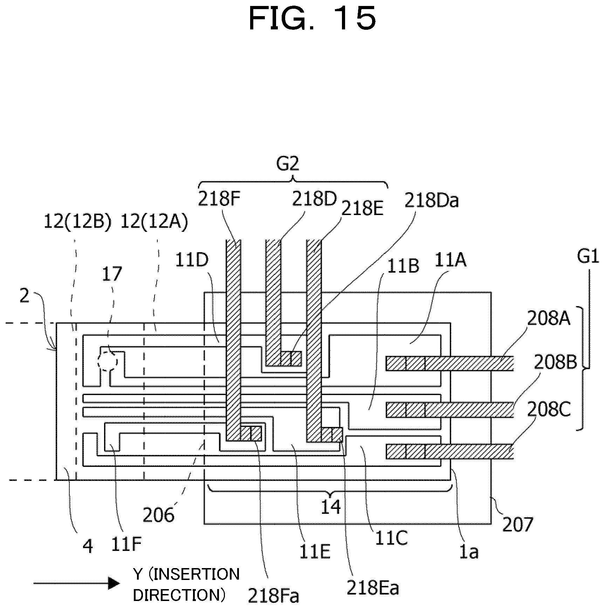

In the first embodiment, the tip portion of each of the connector pins 208D, 208E, and 208F is oriented in a direction (the X direction) perpendicular to the insertion direction of the sensor 1. FIG. 15 is a plan view schematically showing a state where the connecting portion 14 and the receiving portion 207 are fitted with each other according to the third embodiment. The third embodiment includes connector pins 218D, 218E, and 218F in place of the connector pins 208D, 208E, and 208F (FIG. 6A) as connector pins belonging to the second connector pin group G2.

Tip portions 218Da, 218Ea, and 218Fa of the connector pins 218D, 218E, and 218F are bent by approximately 90 degrees relative to a direction (the X direction) in which an arm is extended and are oriented in the insertion direction (the Y direction) . In other words, the tip portions 218Da, 218Ea, and 218Fa of the connector pins 218D, 218E, and 218F are oriented in an opposite direction to the direction in which the tip portions of the connector pins 208A, 208B, and 208C belonging to the first connector pin group G1 are oriented (a direction in which the sensor 1 is separated from the measurement apparatus (referred to as a separation direction or an ejection direction)). With the exception of the configuration of the connector pins 218D, 218E, and 218F, since the configuration of the third embodiment is the same as the configuration of the first embodiment, a description thereof will be omitted.

According to the third embodiment, the tip portions 218Da, 218Ea, and 218Fa of the connector pins 218D, 218E, and 218F are oriented in a same direction (a forward direction) as the insertion direction. Due to this configuration, a friction force when the second connector pin group G2 rubs against the electrode pads 11A to 11F can be reduced as compared to the first embodiment. Therefore, according to the third embodiment, at least one of the second connector pin group G2 (the connector pins 218D, 218E, and 218F: an example of the second conductive portion) and the electrode pads 11A to 11F can be prevented from being damaged by friction created during insertion of the sensor 1. In addition, even during ejection of the sensor 1, the possibility that the second connector pin group G2 and the electrode pads 11A to 11F rub against and damage each other when the sensor 1 moves in an ejection direction that is opposite to the insertion direction can be reduced.

Furthermore, since friction force is reduced, ejection of the sensor 1 can be readily performed. It should be noted that a part of or all of the tip portions 218Da, 218Ea, and 218Fa can be oriented in the ejection direction (a direction opposite to the insertion direction) . In addition, the tip portion of the connector pin 208G for specification determination according to the second embodiment may be bent so as to be oriented in the insertion direction or the ejection direction in a similar manner to the connector pin 218D and the like.

### Fourth Embodiment

A measurement system according to a fourth embodiment will be described. Since the fourth embodiment shares the configuration of the first embodiment, differences will be mainly described below and descriptions of common points will be omitted. As a measurement apparatus according to the fourth embodiment, the measurement apparatuses 200, 200A, and 200B described in the first embodiment can be applied. However, the fourth embodiment differs from the first embodiment in a configuration of connector pins belonging to the second connector pin group G2.

In the first embodiment, respective arm portions of the connector pins 208D, 208E, and 208F belonging to the second connector pin group G2 are oriented in the Y direction of the measurement apparatus 200 or, in other words, a horizontal direction when the measurement apparatus is horizontally arranged (FIG. 7). In the fourth embodiment, a configuration is adopted in which a part of or all of the plurality of conductive portions (the connector pins 208) slopes downward so as to intersect, between a base end portion (the arm portion) and a tip portion thereof, the insertion direction (the Y direction) of the sensor 1.

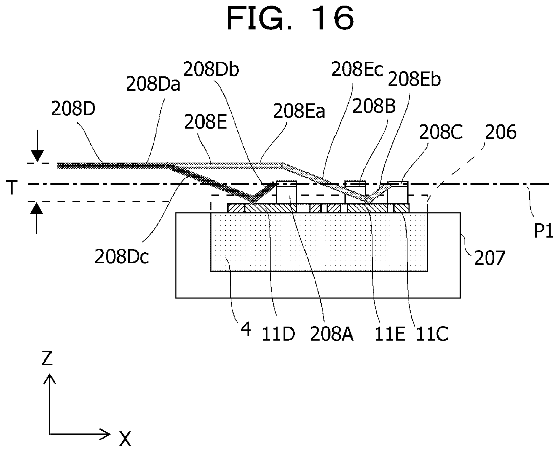

FIG. 16 shows a cross section taken along a dashed-dotted line B-B in FIG. 6A according to the fourth embodiment. The connector pin 208D according to the fourth embodiment has an intermediate portion 208Dc which gradually slopes downward (for example, slopes downward so as to intersect with an XY plane at an angle of 60 degrees or smaller) from an arm portion 208Da toward a tip portion 208Db thereof. In a similar manner, the connector pin 208E also has an intermediate portion 208Ec which extends so as to gradually slope downward from an arm portion 208Ea toward a tip portion 208Eb thereof. Heights of the tip portions 208Db and 208Eb are at lower positions than the arm portions 208Da and 208Ea. It should be noted that, while the connector pin 208F is not illustrated, the connector pin 208F has a similar configuration to the connector pins 208D and 208E. In addition, the connector pins 208 belonging to the first connector pin groups G1 can also be configured in a similar manner.

According to the fourth embodiment, the connector pins 208 have a longer intermediate portion than in the first embodiment. As a result, since the connector pins 208 can be imparted with flexibility (a spring property), the connector pins 208 can be caused to deflect during insertion or ejection of the sensor 1 to prevent the electrode pads 11A to 11F and the connector pins 208 from rubbing against and damaging each other. Although the smaller the angle (in other words, the closer the angle is to 0 degrees) in which the connector pin 208D gradually slopes downward, the greater the effect of preventing mutual damage, the need for a larger space makes it difficult to suppress the size of the sensor. Conversely, although the larger the angle (in other words, the closer the angle is to 60 degrees), the smaller the space required to adopt the configuration, mutual damage is more likely to occur. Therefore, an angle corresponding to the size of the sensor is favorably adopted. For example, a range of 30 to 60 degrees is favorable. The configurations of the first to fourth embodiments described above can be appropriately combined with each other.

## Claims

1. A measurement apparatus for measuring a target substance in a sample adhered to a sensor, the measurement apparatus comprising:
an insertion opening used to insert an inserting portion of the sensor within the measurement apparatus, the inserting portion including a plane having a plurality of electrode pads; and
a plurality of conductive portions configured to contact with the plurality of electrode pads in an inserted state of the sensor indicating a state that the inserting portion of the sensor is inserted within the measurement apparatus,
wherein the plurality of conductive portions includes a first conductive portion group extending in an insertion direction of the sensor, the insertion direction indicating a direction of the sensor when the inserting portion is inserted within the measuring apparatus passing through the insertion opening, and a second conductive portion group extending in a direction intersecting with the insertion direction, and
wherein in the inserted state, the first conductive portion group is configured to contact with electrode pads corresponding to the first conductive portion group among the plurality of electrode pads, and the second conductive portion group is configured to contact with electrode pads other than the electrode pads corresponding to the first conductive portion group among the plurality of electrode pads.

2. The measurement apparatus according to claim 1, wherein each of the plurality of conductive portions has one end and other end, the one end is supported in the measuring apparatus, and the other end is contactable the plurality of electrode pads formed on the plane of the inserting portion in the inserted state, and
wherein in the inserted state, each of the plurality of conductive portions has a farthest portion apart from the plane in the normal direction of the plane, a distance in the normal direction between the plane and the farthest portion is shorter than a given distance.

3. The measurement apparatus according to claim 2, wherein in the inserted state, a level of surface of each of the plurality of electrode pads in the horizontal direction of the plane is same, and
wherein a distance in the normal direction of the plane between the surface and the farthest portion is within 1.5 times a thickness of each of the plurality of conductive portions.

4. The measurement apparatus according to claim 1, wherein in the inserted state, each of the plurality of conductive portions is arranged on a plane that is parallel to the plane included in the inserting portion.

5. The measurement apparatus according to any one of claims 1 to 4, wherein the second conductive portion group are configured to contact with electrode pads other than the contact pads contacting with the first conductive portion group in the inserted state among the plurality of electrode pads.

6. The measurement apparatus according to any one of claims 1 to 5, further comprising a determining portion configured to determine, in the inserted state, whether a first conductive portion in the first conductive portion group and a second conductive portion in the second conductive portion group are in contact with a same electrode among the plurality of contact pads or in contact with two different electrode pads among the plurality of electrode pads.

7. The measurement apparatus according to claim 6, wherein the determining portion is configured to determine whether or not a change in a current occurs by applying a voltage between the first conductive portion and the second conductive portion.

8. The measurement apparatus according to any one of claims 1 to 5, further comprising a determining portion configured to determine, in the inserted state, whether two second conductive portions in the second conductive portion group are in contact with a same electrode pad among the plurality of contact pads or in contact with two different electrode pads among the plurality of electrode pads.

9. The measurement apparatus according to claim 8, wherein the determining portion is configured to determine whether or not a change in a current occurs by applying a voltage between the two second conductive portions.

10. A measurement system, comprising:
a sensor to which a sample is to be adhered; and
a measurement apparatus for measuring a target substance in the sample,
wherein the measurement apparatus includes:
an insertion opening used to insert an inserting portion of the sensor within the measurement apparatus, the inserting portion including a plane having a plurality of electrode pads; and
a plurality of conductive portions configured to contact with the plurality of electrode pads in an inserted state of the sensor indicating a state that the inserting portion of the sensor is inserted within the measurement apparatus,
wherein the plurality of conductive portions includes a first conductive portion group extending in an insertion direction of the sensor, the insertion direction indicating a direction of sensor when the inserting portion is inserted within the measuring apparatus passing through the insertion opening, and a second conductive portion group extending in a direction intersecting with the insertion direction, and
wherein in the inserted state, the first conductive portion group is configured to contact with electrode pads corresponding to the first conductive portion group among the plurality of electrode pads, and the second conductive portion group is configured to contact with electrode pads other than the electrode pads corresponding to the first conductive portion group among the plurality of electrode pads.

11. The measurement apparatus according to any one of claims 1 to 9, wherein a tip portion of conductive portions belonging to the second conductive portion group is oriented in the insertion direction.

12. The measurement apparatus according to any one of claims 1 to 9 and 11, wherein the second conductive portion group is arranged such that, during insertion of the inserting portion, the second conductive portion group and the plurality of electrode pads rub against each other, and, when the inserting portion reaches a given position, the second conductive portion group contacts with electrode pads corresponding to the second conductive portion group.

13. The measurement apparatus according to any one of claims 1 to 9, 11, and 12, wherein at least one of the plurality of conductive portions has an intermediate portion between a base end portion and a tip portion thereof, and wherein the intermediate portion slopes downward so as to intersect the insertion direction.
